# EUROPEAN PATENT APPLICATION

(11) **EP 4 772 121 A1**
(43) Date of publication of application: **08.07.2026**
(21) Application number: 24859835.1
(22) Date of filing: 28.08.2024
(51) Int. Cl.: A61B 18/02

(54) **CRYOTHERAPY DEVICE**

(30) Priority: 28.08.2023 JP 2023138209
(71) Applicant: Gunze Limited, Ayabe-shi, Kyoto 623-8511 (JP); Keio University, Tokyo, 108-8345 (JP)
(72) Inventor: KAMIKYU, Keisuke, Ayabe-shi, Kyoto 623-8511 (JP); MISHUKU, Kazuto, Ayabe-shi, Kyoto 623-8511 (JP); SUZUKI, Shingo, Ayabe-shi, Kyoto 623-8511 (JP); ISHII, Tatsuyuki, Tokyo 160-8582 (JP); KISHI, Kazuo, Tokyo 160-8582 (JP)
(74) Representative: Mewburn Ellis LLP
(86) International application number: PCT/JP2024/030735
(87) International publication number: WO 2025/047805

(57) **Abstract**

This cryotherapy device comprises a head part, a cooling mechanism, and a control unit. The head part has a probe that forms a working surface to be brought close to or into contact with the surface of a skin to be treated. The cooling mechanism cools the probe. The control unit controls the cooling mechanism such that the probe is maintained at a target temperature of 0°C or lower. The probe is configured so as to freeze the tissue of the skin through the working surface and thereby selectively induce the apoptosis of diseased cells including at least nevus cells.

## Description

### Technical Field

The present disclosure relates to a cryotherapy device.

### Background Art

PTL 1 discloses a system having an electrosurgical probe for ablating integumentary tissue or epidermis tissue of a patient. Examples of the tissue to be ablated include abnormal tissue part, nevi, superficial lentigines, malignant melanoma, and malignant tumor tissue.

### Citation List

### Patent Literature

PTL 1: Japanese Translation of PCT Internation Patent Publication No. H11-501555

### Summary of Invention

### Technical Problem

The electrosurgical probe disclosed in PTL 1 includes an active electrode connected to a high frequency voltage source and a return electrode. When a high frequency voltage is applied to the active electrode and the return electrode, the active electrode creates an energy source at the distal tip, and causes necrosis of the tissue that comes into contact with the distal tip or tissue in the vicinity of such tissue. However, destruction of the skin tissue resultant of use of such an electrosurgical probe is invasive, and there are risks of leaving inflammations, scars, scar contractures, and the like. For this reason, a therapy device capable of removing diseased skin cells such as nevus cells while reducing the risks of leaving scars has been awaited.

An object of the present disclosure is to provide a cryotherapy device capable of removing diseased cells including at least nevus cells, while reducing the risks of leaving scars or the like.

### Solution to Problem

A cryotherapy device according to a first aspect of the present disclosure includes a head unit, a cooling mechanism, and a control unit. The head unit includes a probe forming an active surface to be brought closer to or into contact with the surface of the skin of a treatment site. The cooling mechanism cools the probe. The control unit controls the cooling mechanism so that the probe is sustained at a target temperature of 0°C or below. The probe is configured to freeze the tissue of the skin via the active surface so as to selectively cause apoptosis in a diseased cell including at least a nevus cell.

The cryotherapy device according to the first aspect selectively causes, with the use of a probe cooled to 0°C or below, natural death of diseased cells including at least nevus cells (to undergo apoptosis, in which cells are fragmented into small pieces without breaking the cell membranes). In the removal of diseased cells including nevus cells, conventionally, treatment such as invasive surgical ablation, treatment using a laser, treatment using an electric scalpel, and cryotherapy using a cryogenic liquid gas has been practiced. Because these types of treatment induce necrosis (in which the cell membrane breaks and the content of the cells is released) not only of diseased cells but also of non-diseased normal cells in the vicinity of the diseased cells, the treatments often leave scars, scar contractures, and inflammations. The inventors have found out that, as a result of intensive studies, when nevus cells and normal cells are subjected to cooling at 0°C or below, a significantly higher proportion of the nevus cells subsequently reaches apoptosis than that of the normal cells reaching apoptosis. This can be possibly attributed to the nevus cells being more temperature-sensitive than the normal cells. With a cryotherapy device based on this finding, it is possible to cause apoptosis in the diseased cells, which are more temperature-sensitive, preferentially over the normal cells.

Furthermore, in the cryotherapy device according to the first aspect, the active surface of the probe having been cooled to 0°C or below causes the tissue of the skin of the treatment site to freeze. Therefore, it is not always necessary to prepare a low-temperature liquefied gas, such as liquid nitrogen or carbon dioxide, so that the burden of complicated safety management of high-pressure gas is eliminated.

A cryotherapy device according to a second aspect of the present disclosure is the cryotherapy device according to the first aspect, in which the probe further forms a pair of surfaces intersecting with the active surface; the cooling mechanism includes a pair of cooling elements capable of cooling the probe; and each of the pair of cooling elements forms a first surface capable of absorbing heat from the pair of surfaces.

With the cryotherapy device according to the second aspect, the cooling capacity of the probe can be further improved with the cooling elements.

A cryotherapy device according to a third aspect of the present disclosure is the cryotherapy device according to the first or second aspect, in which each of the pair of cooling elements further forms a second surface capable of releasing the heat absorbed from the first surface, and the cooling mechanism further includes a cooling jacket through which a refrigerant capable of absorbing heat of the second surface is circulated.

With the cryotherapy device according to the third aspect, the cooling efficiency of the probe can be further improved.

A cryotherapy device according to a fourth aspect of the present disclosure is the cryotherapy device according to any one of the first to third aspects, in which the head unit further includes: a casing that houses at least a part of each of the probe, the pair of cooling elements, and the cooling jacket; and a thermal insulation material disposed between the casing and the cooling jacket.

With the cryotherapy device according to the fourth aspect, the cooling efficiency of the probe can be further improved.

A cryotherapy device according to a fifth aspect of the present disclosure is the cryotherapy device according to any one of the first to fourth aspects, in which the head unit further includes an imaging device that acquires an image of the diseased cells including at least the nevus cell or vicinity of the diseased cells.

With the cryotherapy device according to the fifth aspect, observations of the conditions of the diseased cells or the vicinity of the diseased cells are made easy even in a case where such cells or the vicinity of such cells are hidden behind the head unit, which makes the visual observations difficult.

A cryotherapy device according to a sixth aspect of the present disclosure is the cryotherapy device according to any one of the first to fifth aspects, in which the control unit controls the cooling mechanism so that the probe is sustained at a target temperature of -30°C or above.

A cryotherapy device according to a seventh aspect of the present disclosure is the cryotherapy device according to any one of the first to sixth aspects, in which the control unit controls the cooling mechanism so that the probe is sustained at a first target temperature of -30°C or above and -25°C or below.

A cryotherapy device according to an eighth aspect of the present disclosure is the cryotherapy device according to any one of the first to seventh aspects, in which the control unit controls the cooling mechanism to keep a time length for which the temperature of the probe is sustained at the first target temperature equal to or shorter than a first time length.

According to the eighth aspect, by causing the control unit to control the cooling mechanism, the time length for which the temperature of the probe is sustained at the first target temperature of -30°C or above and -25°C or below becomes equal to or shorter than the first time length. In this manner, it is possible to avoid freezing of the skin of the treatment site over a length of time longer than necessary.

A cryotherapy device according to a ninth aspect of the present disclosure is the cryotherapy device according to any one of the first to eighth aspects, in which the first time length is one minute.

A cryotherapy device according to a tenth aspect of the present disclosure is the cryotherapy device according to any one of the first to ninth aspects, in which the control unit controls the cooling mechanism so that the probe is sustained at a second target temperature of -4°C or above and 0°C or below.

A cryotherapy device according to an eleventh aspect of the present disclosure is the cryotherapy device according to any one of the first to tenth aspects, in which the control unit controls the cooling mechanism to keep a time length for which the temperature of the probe is sustained at the second target temperature equal to or shorter than a second time length.

According to the eleventh aspect, by causing the control unit to control the cooling mechanism, the time length for which the temperature of the probe is sustained at the second target temperature of -4°C or above and 0°C or below becomes equal to or shorter than the second time length. In this manner, it is possible to avoid freezing of the skin of the treatment site over a length of time longer than necessary.

A cryotherapy device according to a twelfth aspect of the present disclosure is the cryotherapy device according to any one of the first to eleventh aspects, in which the second time length is five minutes.

A cryotherapy device according to a thirteenth aspect of the present disclosure is the cryotherapy device according to any one of the first to twelfth aspects, in which the probe is made of metal.

According to the thirteenth aspect, it is possible to cool the probe efficiently.

A cryotherapy device according to a fourteenth aspect of the present disclosure is the cryotherapy device according to any one of the first to thirteenth aspects, in which the head unit includes a temperature sensor that detects a temperature of the probe, and the control unit feedback-controls the temperature of the probe based on an output signal from the temperature sensor.

According to the fifteenth aspect, the probe is sustained in the temperature range with higher accuracy.

A cryotherapy device according to a sixteenth aspect of the present disclosure is the cryotherapy device according to any one of the first to fifteenth aspects, in which the head unit further includes a thermal insulation portion forming a surface surrounding the active surface.

According to the fifteenth aspect, it is possible to suppress undesirable effects on the cells around the tissue that is in the vicinity of or in contact with the active surface.

A cryotherapy device according to a sixteenth aspect of the present disclosure is the cryotherapy device according to any one of the first to fifteenth aspects, in which the cooling mechanism includes a cooling element that cools the probe, and a chiller that cools the cooling element; and the probe, the cooling element, and the chiller are connected in an order listed herein, with reference to the active surface.

According to the sixteenth aspect, by using the cooling element connected to the probe, as a part of the cooling mechanism, the entire cryotherapy device can be reduced in size.

A cryotherapy device according to a seventeenth aspect of the present disclosure is the cryotherapy device according to any one of the first to sixteenth aspects, in which the control unit controls the cooling mechanism in accordance with at least two different operation conditions each including a preset combination of a target temperature for the probe and a time length for which the target temperature is to be sustained; and the cryotherapy device further comprises a condition designation unit that allows a user to designate one of the at least two different operation conditions.

According to the seventeenth aspect, it is possible to cause the cryotherapy device to operate selectively between at least two operation conditions. In this manner, more effective treatment can be provided depending on the diseased cells. Advantageous Effects of Invention

According to the present disclosure, a cryotherapy device capable of removing diseased cells including at least nevus cells while reducing the risks of leaving scars or the like is provided.

### Brief Description of Drawings

[Fig. 1] Fig. 1 is a schematic diagram illustrating an exemplary configuration of a cryotherapy device according to a first embodiment.
[Fig. 2] Fig. 2 is a schematic cross-sectional view illustrating an exemplary configuration of a head unit.
[Fig. 3] Fig. 3 is a plan view of the head unit as viewed from the side of an active surface.
[Fig. 4] Fig. 4 is a block diagram illustrating an electrical configuration of a control unit.
[Fig. 5] Fig. 5 is a schematic diagram illustrating an exemplary configuration of a cryotherapy device according to a second embodiment.
[Fig. 6A] Fig. 6A is an external perspective view of a head unit according to the second embodiment.
[Fig. 6B] Fig. 6B is a schematic cross-sectional view illustrating an exemplary configuration of a head unit according to the second embodiment.
[Fig. 7] Fig. 7 is a flowchart illustrating an example of the sequence of an operation of the cryotherapy device.
[Fig. 8A] Fig. 8A is an external perspective view illustrating an exemplary configuration of a probe according to the second embodiment.
[Fig. 8B] Fig. 8B is an external perspective view illustrating an exemplary configuration of the probe according to the second embodiment.
[Fig. 8C] Fig. 8C is an external perspective view illustrating an exemplary configuration of the probe according to the second embodiment.
[Fig. 8D] Fig. 8D is an external perspective view illustrating an exemplary configuration of the probe according to the second embodiment.
[Fig. 9A] Fig. 9A is a side view of a probe used in an experiment.
[Fig. 9B] Fig. 9B is a side view of a probe having compatibility with the probe in Fig. 8A.
[Fig. 10] Fig. 10 is a micrograph showing results of experiments (Example 1 and Comparative Example) carried out by the inventors.
[Fig. 11] Fig. 11 is a micrograph showing the result of an experiment (Example 2) carried out by the inventors.
[Fig. 12] Fig. 12 is a micrograph showing the result of an experiment (Example 3) carried out by the inventors.
[Fig. 13] Fig. 13 is a micrograph showing the result of an experiment (Example 4) carried out by the inventors.
[Fig. 14] Fig. 14 is a micrograph showing the result of an experiment (Example 5) carried out by the inventors.
[Fig. 15] Fig. 15 is a micrograph showing the result of an experiment (Example 6) carried out by the inventors.
[Fig. 16] Fig. 16 is a micrograph showing the result of an experiment (Example 7) carried out by the inventors.

### Description of Embodiments

A cryotherapy device according to some embodiments of the present disclosure will now be explained with reference to drawings. In the drawings described below, some components may be omitted as appropriate, for the convenience of description. Furthermore, the dimensions of the components illustrated in the drawings described below may not necessarily correspond to the dimensions of the actual components.

### <1-1. Configuration of cryotherapy device according to first embodiment>

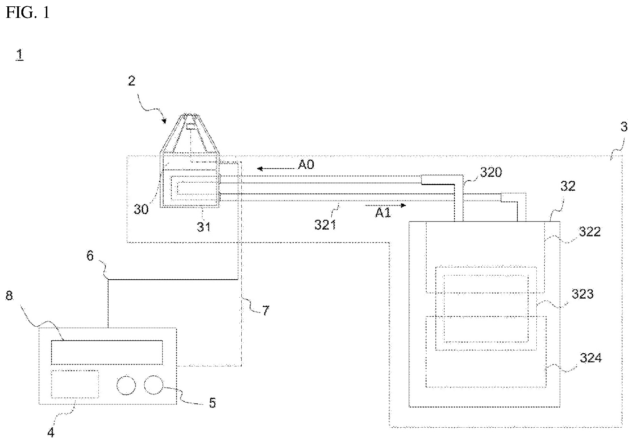

Fig. 1 is a schematic diagram illustrating an exemplary configuration of a cryotherapy device 1 according to a first embodiment. The cryotherapy device 1 is a device for providing treatment on diseased cells including at least nevus cells (including giant nevus cells), while suppressing risks of scars, scar contractures, and inflammations, and main intended users are healthcare workers. The diseased cells to be treated by the cryotherapy device 1 are not limited to any particular cells, as long as such cells are visible on the skin surface and have higher temperature sensitivity than that exhibited by normal cells, and the diseased cells also include pigmented cells (melanocytes) and inflammatory cells, as well as nevus cells. Examples of the disease to be treated include acanthosis nigricans, malignant melanoma (melanoma), senile lentigines, flat nevi, acquired melanocytosis (ADM), seborrheic keratosis (senile warts), neurofibroma, cutaneous fibroma, hypertrophic scar, and keloid.

As illustrated in Fig. 1, the cryotherapy device 1 includes a head unit 2, a cooling mechanism 3 connected to the head unit 2, a control unit 4, and a condition designation unit 5, and each of these units operates in a manner to be described later, by receiving supply of power from a power source (not illustrated). Each of these units will now be explained.

### [Head unit]

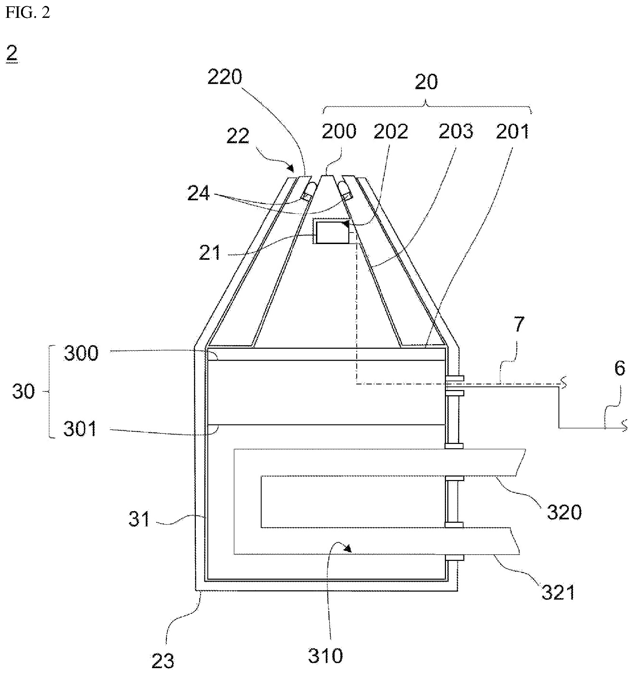

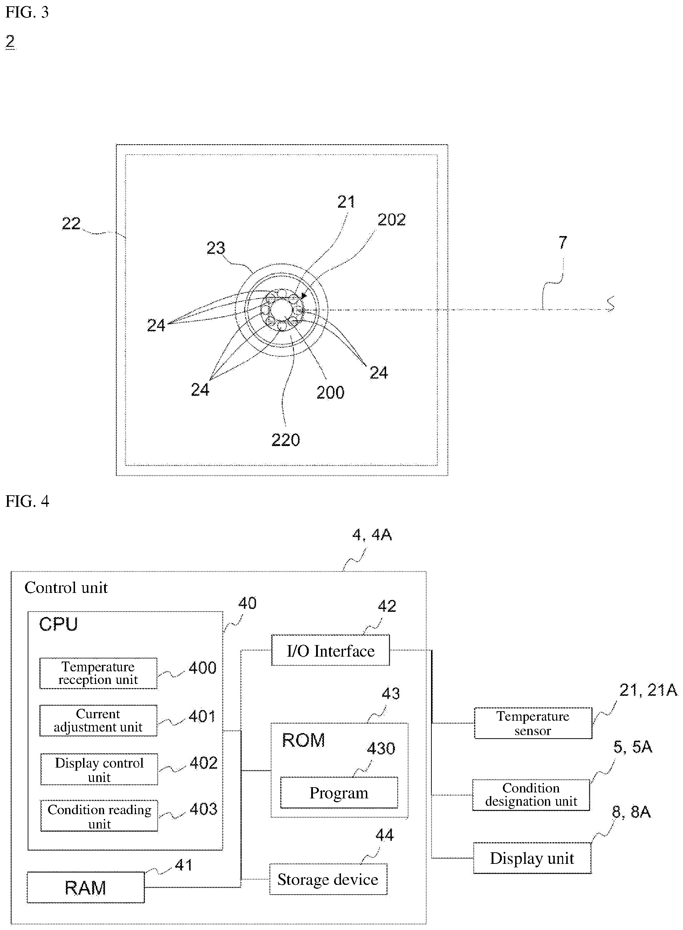

Fig. 2 is a schematic cross-sectional view illustrating an exemplary configuration of the head unit 2, and Fig. 3 is a plan view of the head unit 2 as viewed from the side of an active surface 200. The head unit 2 is a part to be gripped by a user during treatment, and exerting an effect on the skin surface of a treatment site. The head unit 2 includes a probe 20, a temperature sensor 21, a thermal insulation portion 22, an illumination 24, and a casing 23. The casing 23 houses at least a part of the probe 20, the temperature sensor 21, the illumination 24, and the thermal insulation portion 22, and houses at least a part of a cooling element 30 and a heat-releasing member 31 of the cooling mechanism 3, which will be described later. The material with which the casing 23 is formed is not limited to any particular material, but a material that is less thermally conductive than the material of the probe 20 is preferable, and examples of such a material include a hard resin. The casing 23 may be configured as a gripping portion for enabling a user to grip and to operate the probe 20.

The probe 20 is a member integrally made of metal, and forms an active surface 200 to be brought into contact with the skin surface of the treatment site. Hereinafter, the side of the active surface 200 of the probe 20 is defined as a distal-end side of the probe, and the side facing the opposite side of the active surface 200 is defined as a proximal-end side of the probe 20. The active surface 200 according to the embodiment is a flat surface having a circular shape, as viewed from the distal-end side, but the shape of the active surface 200 is not limited to any particular shape, and may be changed as appropriate. When the active surface 200 has a circular shape, or when the active surface 200 has a square shape, the diameter of the circle or the length of one side of the square is preferably 1 mm or more and 10 mm or less. The active surface 200 may be exposed from the casing 23.

On the proximal-end side of the probe 20 according to the embodiment, a base 201 having a substantially flat plate-like shape is provided. The base 201 is connected to the cooling element 30, with the proximal-end side surface of the base 201 facing a first surface 300 of the cooling element 30. The base 201 and the cooling element 30 may be connected by fastening with a screw, for example, and thermally conductive grease or the like may be placed therebetween. From the viewpoint of cooling the probe 20 efficiently, it is preferable for the base 201 to have a shape and dimensions in common with those of the first surface 300 of the cooling element 30, as viewed from the proximal-end side, and for the proximal-end side surface of the base 201 and the first surface 300 to entirely overlap each other. In this embodiment, because the first surface 300 has a substantially rectangular shape, the base 201 also has a substantially rectangular shape, in a view from the proximal-end side. Therefore, the probe 20 according to the embodiment has a substantially truncated conical outer appearance that rises from the base 201 in a substantially circular shape, that extends such that the diameter thereof becomes smaller toward the distal-end side, and that is continuous to the perimeter of the active surface 200. However, the outer shape of the probe 20 is not limited thereto, and may be changed as appropriate, on the basis of the shape of the active surface 200 or the cooling element 30.

The metal forming the probe 20 is not limited to any particular metal, as long as the metal is not harmful to human bodies, and examples of such a metal include medical metal materials and aluminum. It is possible for these metals to be surface-treated as long as the thermal conductivity of the active surface 200 is not affected thereby.

The temperature sensor 21 is disposed in a space 202 formed inside the probe 20. The space 202 in the embodiment extends from the circumferential side surface 203 of the probe 20 to the inside of the probe 20, and is a space one side of which is closed. The circumferential side surface 203 of the probe 20 herein is a surface extending continuously between the perimeter of the active surface 200 and the base 201. In order to minimize the difference between a temperature detected by the temperature sensor 21 and a temperature of the active surface 200, the space 202 is preferably provided in the vicinity of the active surface 200. The temperature sensor 21 detects the temperature of the probe 20 and transmits output signals representing the detected temperature to the control unit 4, to be described later, from moment to moment. As will be described later, the control unit 4 feedback-controls the cooling mechanism 3 on the basis of the output signals thus transmitted. The output signals may be transmitted over the wire via a communication line 7, or wirelessly. The temperature sensor 21 is not limited to any particular sensor as long as the sensor can detect the temperature of the probe 20, and any known sensor such as a resistance thermometer, a thermocouple, a thermistor sensor, or a thermostat may be used.

The thermal insulation portion 22 is a part disposed in a manner surrounding the probe 20 inside the casing 23, and forming a surface 220 surrounding the active surface 200. In the embodiment, the surface 220 is provided flush with the active surface 200. The thermal insulation portion 22 can suppress undesirable effects on the cells around the tissue with which the active surface 200 is in contact. One example of the undesirable effects is an effect by which the apoptosis is induced in the normal cells outside the treatment site because the air surrounding the normal cells is unintentionally cooled by the active surface 200.

As the material with which the thermal insulation portion 22 is formed, there is no limitation to any particular material, and any known thermal insulation material may be used as long as the material is less thermally conductive than the metal of the probe 20 and is not harmful to human bodies. In the embodiment, the thermal insulation portion 22 is made of polystyrene foam.

The probe 20 according to the embodiment further includes the illumination 24. There is no particular limitation to the illumination 24 as long as the illumination can illuminate the skin surface facing the active surface 200, and any known light source such as a laser light source or an LED may be used. The illumination 24 may be disposed in a manner surrounding the active surface 200 so that the skin surface is irradiated with the light emitted from the space between the active surface 200 and the surface 220, for example. With this, the illumination 24 provides an aid in bringing the active surface 200 closer to or into contact with the skin surface of the treatment site. Note that the illumination 24 and the temperature sensor 21 are connected to a power source, as appropriate, using a conductive wire (not illustrated), for example.

### [Cooling mechanism]

The cooling mechanism 3 includes the cooling element 30, the heat-releasing member 31, and a chiller 32. As described earlier, in the embodiment, because the cooling element 30 and the heat-releasing member 31 are housed inside the casing 23, the probe 20, the cooling element 30, and the heat-releasing member 31 are connected in the order listed herein, with reference to the active surface 200, and are integrated by the casing 23. The chiller 32 is connected to the heat-releasing member 31 via a first pipe 320 and a second pipe 321. The heat-releasing member 31, the first pipe 320, the second pipe 321, and the chiller 32 together form a refrigerant circulation path by which the heat of the cooling element 30 is removed. Description thereof will follow.

The cooling element 30 is an element capable of cooling the probe 20 from the side of the first surface 300, and in the embodiment, a Peltier element is used as the cooling element 30. For this purpose, a conductive wire 6 via which a direct current is passed is connected to the cooling element 30 according to the embodiment. The conductive wire 6 is connected as a pair to the one cooling element 30, and the opposite side of the pair of conductive wires 6 is connected to the control unit 4. The control unit 4 applies a direct current to the cooling element 30 so that the first surface 300 serves as a heat absorbing side and a second surface 301 serves as a heat generating side. As will be described later, the control unit 4 controls the behavior of the cooling element 30 by controlling the direct current to be passed through the cooling element 30 so that the probe 20 is sustained at a predetermined target temperature of 0°C or below.

The heat-releasing member 31 is a member for releasing the heat on the side of the second surface 301 of the cooling element 30. The heat-releasing member 31 according to the embodiment has a substantially flat plate-like outer shape. The heat-releasing member 31 is connected to the cooling element 30 with the surface on one side of the heat-releasing member 31 facing the second surface 301 of the cooling element 30. The heat-releasing member 31 and the cooling element 30 may be connected by fastening with a screw, for example, and thermally conductive grease or the like may be placed therebetween. From the viewpoint of efficiently releasing the heat of the cooling element 30, preferably, the surface of the heat-releasing member 31 and the second surface 301 entirely overlap each other.

In the heat-releasing member 31, a channel 310 through which the refrigerant is passed is formed. The channel 310 is a continuous channel in which one end of the channel 310 is connected to the first pipe 320 and the other end is connected to the second pipe 321. The shape of the channel 310 is not limited to any particular shape, and may be changed as appropriate, in consideration of cooling efficiency or the like. The channel 310 according to the embodiment is configured so that the refrigerant cooled by the chiller 32 flows through the first pipe 320, and the refrigerant having flown through the channel 310 goes out from the second pipe 321. The heat-releasing member 31 and the refrigerant are not limited to any particular heat-releasing member and refrigerant, as long as heat of the cooling element 30 can be removed, and any known water-cooled heat sink and coolant may be used, for example.

The chiller 32 is a device for passing the refrigerant through the first pipe 320, the second pipe 321, and the heat-releasing member 31, and any known cooling water circulation device may be used. The chiller 32 includes a water tub 322 where the refrigerant to be circulated is stored, a cooler 323 for cooling the refrigerant in the water tub 322, and a heat exchanger unit 324. The refrigerant in the water tub 322 is sent out to the heat-releasing member 31 through the first pipe 320, by a pump or the like (not illustrated) (see the arrow A0 in Fig. 1). The refrigerant having the heat absorbed by the heat-releasing member 31 is returned to the water tub 322 via the second pipe 321 (see the arrow A1 in Fig. 1). The heat of the refrigerant having returned is removed by the cooler 323. In this manner, the refrigerant is cooled, is sent out again from the water tub 322 to the first pipe 320 and the heat-releasing member 31, and repeats the circulation. The cooler 323 is not limited to any particular cooler, and for example, any known cooling coil or the like internal of which chiller-side refrigerant is passed may be used.

The heat exchanger unit 324 is a device that circulates the chiller-side refrigerant while removing and releasing the heat of the chiller-side refrigerant flowing inside the cooler 323. The heat exchanger unit 324 is not limited to any particular heat exchanger, but any known heat exchange circuit including a compressor for compressing the chiller-side refrigerant and raising the temperature, a condenser, a fan, and an expansion valve may be used. The chiller-side refrigerant repeats absorbing the heat from the refrigerant in the water tub 322 via the cooler 323, and releasing the heat, by circulating through the heat exchange circuit in the heat exchanger unit 324. The heat exchanger unit 324 according to the embodiment is an air-cooled heat exchanger, and is configured to suction and discharge the air using a fan.

### [Control unit]

Fig. 4 is a block diagram illustrating an electrical configuration of the control unit 4. The control unit 4 may be configured using a general-purpose computer unit such as a micro-controller unit (MCU). The control unit 4 according to the embodiment includes a central processing unit (CPU) 40, a random access memory (RAM) 41, an I/O interface 42, a read only memory (ROM) 43, and a rewritable nonvolatile storage device 44. These elements are electrically connected to each other via a bus line, and are enabled to communicate with each other. In the ROM 43, a program 430 is written via network communication, via communication with another computer, or via a nonvolatile computer-readable medium. In this manner, the control unit 4 according to the embodiment is manufactured. The program 430 may be written in the storage device 44 instead of the ROM 43.

The CPU 40 virtually operates as a temperature reception unit 400, a current adjustment unit 401, a display control unit 402, and a condition reading unit 403, by reading and executing the program 430. The operation of each of these units 400 to 403 will be described later. The RAM 41 is used for the calculation performed by the CPU 40, as appropriate.

The storage device 44 is implemented as a hard disk, an optical disk, or a flash memory, for example. The storage device 44 stores therein at least two different predetermined operation conditions for the cryotherapy device 1. The operation condition is a preset combination of a target temperature for the probe 20 and a time length for which the target temperature is to be sustained, with the aim of providing more appropriate and effective treatment in a manner suitable for the condition of the tissue in the treatment site. The time length for which the target temperature is to be sustained can be also said to be a time length for which the active surface 200 of the probe 20 having the target temperature is kept in contact with the skin surface. In the embodiment, a first time length for sustaining a first target temperature of -30°C or above and -25°C or below is determined, and a second time length for sustaining a second target temperature of -4°C or above and 0°C or below is determined. Hereinafter, the combination of the first target temperature and the first time length will be referred to as a first operation condition, and a combination of the second target temperature and the second time length will be referred to as a second operation condition. According to the study of the inventors, the first time length is preferably one minute, and more preferably 15 seconds. Alternatively, the first time length is preferably a predetermined time length shorter than one minute, and more preferably a predetermined time shorter than 15 seconds. The second time length is preferably five minutes, and more preferably 60 seconds. Alternatively, the second time length is preferably a predetermined time length shorter than five minutes, and more preferably a predetermined time length shorter than 60 seconds.

The I/O interface 42 functions as an interface for executing data input/output with respect to the temperature sensor 21, the condition designation unit 5, the display unit 8, and other computers, over the wire or wirelessly. The condition designation unit 5 and the display unit 8 are electrically connected to the control unit 4, and in the embodiment, the condition designation unit 5, the display unit 8, as well as the control unit 4, are housed in the same housing and together form a controller unit. The condition designation unit 5 and the display unit 8 will now be explained.

### [Condition designation unit]

The condition designation unit 5 is a part operated by the user to designate the operation condition to the control unit 4, by selecting one of the at least two different operation conditions. The configuration of the condition designation unit 5 is not limited to any particular configuration, and may include a push button, a switching lever, a dial, a keyboard, a touch panel, and a voice recognition module, for example. The operation condition may be designated by, for example, an operation of entering a number or a symbol assigned to a selected operation condition via the condition designation unit 5, an operation of selecting the number or the symbol via the condition designation unit 5, or an operation of entering a voice for identifying the operation condition to the condition designation unit 5. Note that the condition designation unit 5 may also be configured as a part operated by the user in entering other instructions to the control unit 4, in addition to the designation of the operation condition.

### [Display unit]

The display unit 8 may be configured as a liquid crystal display, an organic EL display, a plasma display, and a liquid crystal element, for example, and, when configured as a touch panel display, the display unit 8 may also serve as the condition designation unit 5. The display unit 8 displays various types of information for the user of the cryotherapy device 1. The information to be displayed may be, for example, information on the operation condition designated by the user, or timer information for notifying the lapse of the first time length or the second time length in real time. The operation of the display unit 8 is controlled by the display control unit 402 of the control unit 4.

Other components, such as a speaker outputting sound or voice to the user on the basis of a command from the control unit 4, may be added to the controller unit, as appropriate.

### <1-2. Configuration of cryotherapy device according to second embodiment>

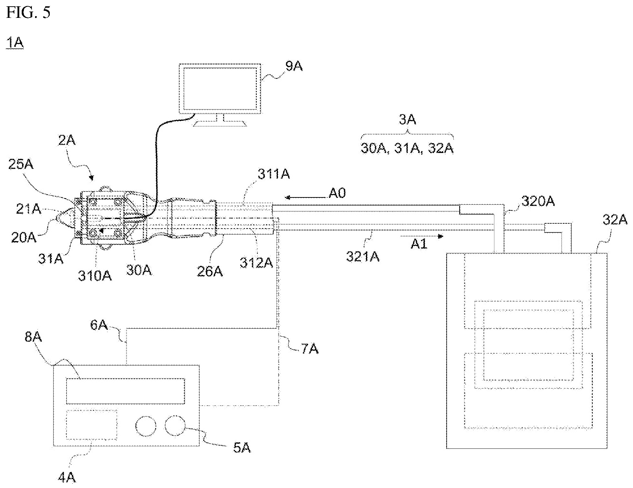

Fig. 5 is a schematic diagram illustrating an exemplary configuration of a cryotherapy device 1A according to a second embodiment. The cryotherapy device 1A includes a head unit 2A, a cooling mechanism 3A connected to the head unit 2A, a control unit 4A, and a condition designation unit 5A, similarly to the cryotherapy device 1, but is different from the cryotherapy device 1 mainly in the configuration of the head unit 2A. The configurations of the cryotherapy device 1A different from that of the cryotherapy device 1 will be mainly explained, and the description of the configurations in common with the cryotherapy device 1 will be omitted as appropriate.

### [Head unit]

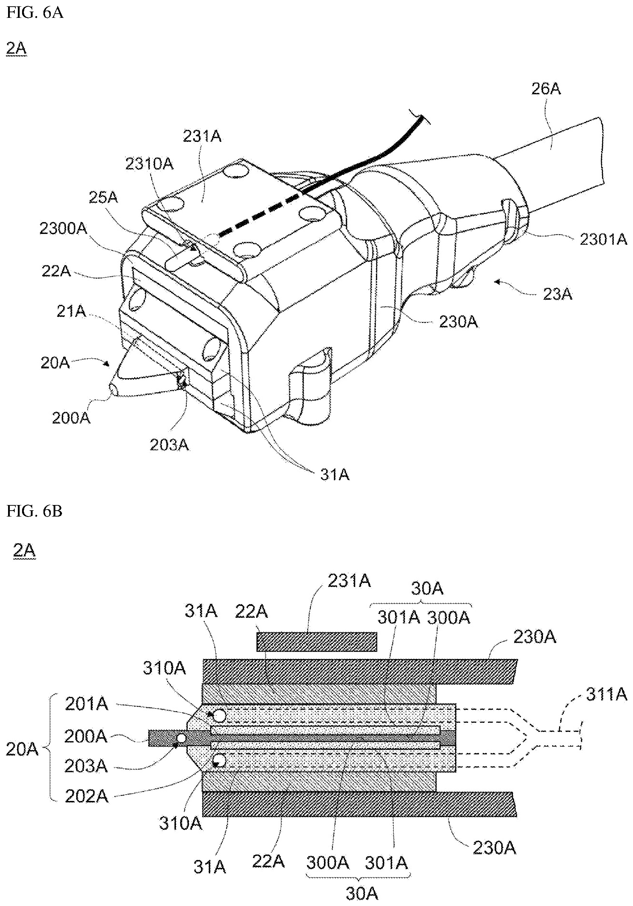

Fig. 6A is an external perspective view of the head unit 2A, and Fig. 6B is a schematic cross-sectional view of an exemplary configuration of the head unit 2A. The head unit 2A includes a probe 20A, a temperature sensor 21A, a thermal insulation material 22A, and a casing 23A. The casing 23A includes a main body 230A and a cover 231A, and is made of, for example, a hard resin, in the same manner as the casing 23. The hard resin is preferably an engineering plastic having excellent heat resistance, such as polyoxymethylene (POM). The main body 230A has a distal-end portion 2300A delineating an opening, and a proximal end 2301A connected to a sleeve member 26A. The sleeve member 26A will be described later. The main body 230A houses at least a part of the probe 20A, the temperature sensor 21A, and the thermal insulation material 22A, and houses at least a part of a pair of cooling elements 30A and a pair of cooling jackets 31A, which are to be described later. In the main body 230A, the pair of cooling elements 30A is disposed in a manner sandwiching the probe 20A, and the pair of cooling jackets 31A is disposed in a manner further sandwiching the pair of cooling elements 30A from the outer side. The thermal insulation material 22A is disposed between the pair of cooling jackets 31A and the main body 230A.

The cover 231A is a member that can be externally attached to the outer surface of the main body 230A. The cover 231A has a holder groove 2310A that holds an imaging device 25A, which will be described later. By fixing the cover 231A to the main body 230A in the orientation where the holder groove 2310A faces the main body 230A, the imaging device 25A can be fixed to the head unit 2A and used as one unit.

The probe 20A is a member integrally made of metal, similarly to the probe 20. The probe 20A is different from the probe 20 in having a substantially plate-like shape. Specifically, the probe 20A at least forms an active surface 200A and a pair of surfaces 201A and 202A intersecting with the active surface 200A. The two surfaces "intersecting" each other means that the normal lines of the respective surfaces intersect each other. In the embodiment, the pair of surfaces 201A and 202A are parallel with each other, and form the front surface and the rear surface of the probe 20A having a substantially plate-like shape. Note that either one of the pair of surfaces 201A and 202A may be the obverse surface (or the reverse surface). The pair of surfaces 201A and 202A face respective first surfaces 300A of a pair of cooling elements 30A, to be described later. The first surfaces 300A are surfaces capable of absorbing heat from the pair of the surfaces 201A and 202A, respectively. The probe 20A preferably has a shape the width of which becomes smaller toward the distal end. With this, it is possible to provide the active surface 200A with a size suitable for coming into contact with general nevus cells, and to provide the pair of surfaces 201A and 202A with a sufficient size to be cooled by the cooling elements 30A.

The active surface 200A is a circular flat surface as viewed from the distal-end side of the probe 20A, and intersects orthogonally with the pair of surfaces 201A and 202A. The perimeter of the active surface 200A may be separated from the perimeters of the pair of surfaces 201A and 202A. In other words, the active surface 200A may be configured to be continuous with the pair of surfaces 201A and 202A via another surface. As to the shape and size of the active surface 200A, the description of the active surface 200 is applicable. In the embodiment, a distal-end portion of the probe 20A including the active surface 200A protrudes outward from the distal-end portion 2300A of the main body 230A. Inside the distal-end portion of the probe 20A, a space 203A is formed. In the space 203A, the temperature sensor 21A is incorporated. The temperature sensor 21A has a configuration in common with the temperature sensor 21.

The thermal insulation material 22A is a member for suppressing the transfer of heat from the pair of cooling jackets 31A to the main body 230A. The thermal insulation material 22A may be configured in any manner as long as the heat exchange between the pair of cooling jackets 31A and the main body 230A is inhibited. Preferably, the thermal insulation material 22A is disposed in a manner surrounding the pair of cooling jackets 31A from outside. As a material with which the thermal insulation material 22A is formed, any material may be used, without limitation to any particular material, as long as the material has a lower thermal conductivity than the material of the cooling jacket 31A and has sufficient heat resistance, and may be any of a mineral-based material, a resin-based material, and a naturally-derived material. Among these materials, in terms of processability, a resin-based foam such as hard urethane foam, phenol foam, polystyrene foam, or polyester foam is preferable.

A conductive wire 6A and a communication line 7A have configuration in common with the conductive wire 6 and the communication line 7, respectively. The conductive wire 6A and the communication line 7A according to the embodiment are passed through the main body 230A, and extend into the sleeve member 26A via a through-hole (not illustrated) provided to the proximal end 2301A. In the sleeve member 26A according to the embodiment, refrigerant tubes 311A and 312A connected to the pair of cooling jackets 31A, as well as the conductive wire 6 and the communication line 7, are enclosed. The refrigerant tubes 311A and 312A will be described later. The sleeve member 26A is not limited to any particular member, and a hose-shaped member such as a known spiral tube or corrugated tube may be used, for example. When the temperature sensor 21A is capable of transmitting an output signal wirelessly, the communication line 7A may be omitted, similarly to the cryotherapy device 1.

The imaging device 25A is installed for the purpose of acquiring an image of diseased cells including at least nevus cells or vicinity of the diseased cells. The imaging device 25A is not limited to any particular imaging device, as long as the imaging device can capture an image of an object, and may be configured as a general-purpose camera, for example. The imaging device 25A may be connected to a general-purpose display 9A. With this, the user can confirm the image captured by the imaging device 25A on the display 9A in real time.

### [Cooling mechanism]

The cooling mechanism 3A includes the pair of cooling elements 30A, the pair of cooling jackets 31A, and a chiller 32A, and cools the probe 20A with the pair of cooling elements 30A and releases the heat of the pair of cooling elements 30A. As each of the cooling elements 30A, a Peltier element forming a first surface 300A and a second surface 301A is used, in the same manner as in the cooling elements 30. The control unit 4A applies a direct current to each of the cooling elements 30A in such a manner that the first surface 300A of the cooling element 30A serves as the heat absorbing side and the second surface 301A serves as the heat generating side.

Each of the pair of cooling jackets 31A has a substantially plate-like outer shape. The cooling jackets 31A are disposed in a manner facing the second surfaces 301A, respectively, and in a manner by which the cooling elements 30A are sandwiched thereby with the surface 201A and the surface 202A of the probe 20A, respectively. Inside each of the cooling jackets 31A, a channel 310A through which a refrigerant capable of absorbing the heat of the second surface 301A circulates is formed. In the embodiment, on the proximal-end side surface of each of the cooling jackets 31A, an inlet (not illustrated) through which the refrigerant goes into the channel 310A and an outlet (not illustrated) through which the refrigerant goes out of the channel 310A are formed. For example, the channel 310A may have a shape extending from the inlet toward the distal-end side of the cooling jacket 31A, turning around on the distal-end side, and extending to the outlet. The inlet of each of the cooling jackets 31A is connected to a first pipe 320A via the refrigerant tube 311A that branches into two on the upstream side of the inlet. The outlet of each of the cooling jackets 31A is connected to the second pipe 321A via the refrigerant tube 312A that branches into two on the downstream side of the inlet. The pair of cooling jackets 31A according to the embodiment is made of aluminum, without limitation thereto. The other elements of each of the cooling jackets 31A and the refrigerant have configurations in common with the heat-releasing member 31 and the refrigerant according to the first embodiment, respectively.

The pair of cooling jackets 31A, the refrigerant tube 311A, the refrigerant tube 312A, the first pipe 320A, the second pipe 321A, and the chiller 32A together form a refrigerant circulation path by which the heat of the pair of cooling elements 30A is removed. The first pipe 320A, the second pipe 321A, and the chiller 32A have configurations that are in common with the first pipe 320, the second pipe 321, and the chiller 32, respectively.

### [Control unit and the like]

The control unit 4A and the condition designation unit 5A have configuration in common with the control unit 4 and the condition designation unit 5, respectively. The display unit 8A is electrically connected to the control unit 4A, and forms a controller unit with the control unit 4 and the condition designation unit 5. The controller unit may have a configuration in common with the controller unit according to the first embodiment. Note that the display unit 8A may have a configuration in common with the display unit 8, or may also serve as the display 9A.

### <2. Operation of cryotherapy device>

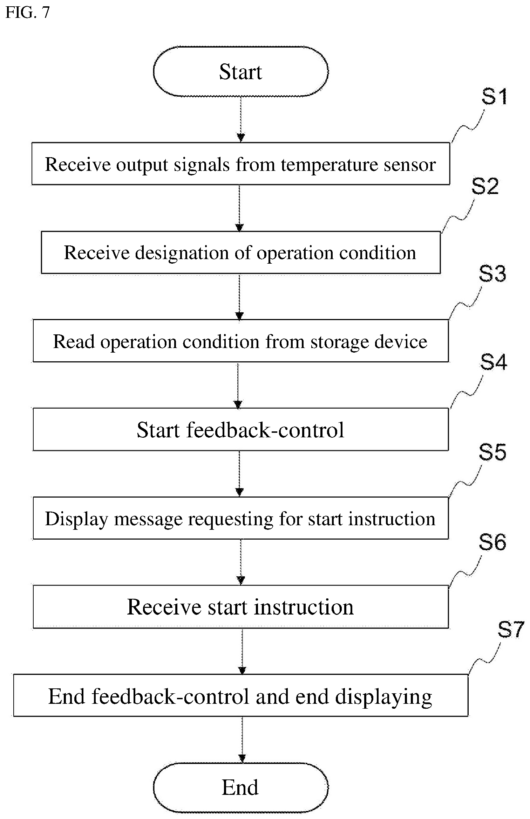

Fig. 7 is a flowchart illustrating an example of the sequence of the operation of the cryotherapy devices 1 and 1A. The operation illustrated in Fig. 7 is performed, for example, after the power supplies of the cryotherapy devices 1 and 1A are turned on, and the refrigerant in the refrigerant circulation path is sufficiently cooled. Hereinafter, the operation of the cryotherapy device 1 will be described as an example, but this same description applies to the operation of the cryotherapy device 1A.

To begin with, the temperature reception unit 400 sequentially receives time-series output signals from the temperature sensor 21 (step S1). The temperature reception unit 400 converts the received output signals into temperature data as necessary, and temporarily stores the temperature data in the RAM 41 or in the storage device 44.

The control unit 4 then receives a designation of the operation condition from the user (step S2). The user can select one of the first operation condition and the second operation condition described above, and then designate the selected operation condition to the control unit 4 by operating the condition designation unit 5.

The condition reading unit 403 then reads information on the operation condition (target temperature and time length) from the storage device 44, on the basis of the received designation of the operation condition (step S3). In step S3, in addition thereto, the display control unit 402 may also cause the display unit 8 to display the designated operation condition and a message to confirm with the user whether this operation condition is really as intended by the user. In this case, the cryotherapy device 1 may be configured to permit the user to confirm the operation condition displayed on the display unit 8, or receive a re-designation of the operation condition by operating the condition designation unit 5.

Once the operation condition is confirmed in step S3, the current adjustment unit 401 and the temperature reception unit 400 start feedback control of the direct current flowing through the cooling element 30 (step S4). Specifically, on the basis of the latest output data received from the temperature sensor 21 by the temperature reception unit 400, the current adjustment unit 401 adjusts the magnitude of the direct current flowing through the cooling element 30 in such a manner that the probe 20 is sustained at the target temperature currently specified in the operation condition having been designated.

Once the accuracy of the temperature control using the feedback control in step S4 stabilizes within a certain range, the next step S5 is executed. In step S5, the display control unit 402 causes the display unit 8 to display a message prompting the user to issue a start instruction.

The user who has confirmed the message operates the condition designation unit 5 and instructs the control unit 4 to start the treatment. At the same time, the user brings the active surface 200 into contact with or close to the skin surface of the treatment site by gripping the head unit 2, for example. The control unit 4 thus receives the instruction for starting the treatment, and starts measuring the time length as specified in the current operation condition having been designated (step S6).

The execution of step S7 is triggered when the time length determined by the current operation condition having been designated has elapsed. In step S7, the current adjustment unit 401 ends the feedback control started in step S4, and stops applying the direct current to the cooling element 30. The display control unit 402 also causes the display unit 8 to display a message notifying that the time length specified in the operation condition has expired. Additionally or alternatively, the control unit 4 may be configured to emit a sound or a voice for notifying that the time length has expired, via a speaker (not illustrated).

### <3. Features>

The cryotherapy devices 1 and 1A according to the embodiments described above are configured to, on the basis of the findings of the inventors, freeze skin cells with the probes 20 and 20A being sustained at a target temperature of -30°C or above and 0°C or below, and to selectively cause apoptosis in the nevus cells. As described above, the cryotherapy devices 1 and 1A are based on a mechanism that is different from surgical ablation, laser cauterization, ablation with the use of an electric scalpel, and cryotherapy with a low-temperature liquefied gas, in which necrosis occurs in normal cells as well as in nevus cells. With the cryotherapy device 1, because damage of normal cells is suppressed, scars, scar contractures, and inflammations are less likely to be formed even after nevus cells are removed, and therefore, treatment giving more consideration to the appearance can be provided.

In the cryotherapy devices 1 and 1A according to the embodiments described above, the probes 20 and 20A made of metal are cooled using Peltier elements. Therefore, the head units 2 and 2A can be reduced in size, so that the user operability is improved. Further, relatively highly accurate temperature control of the probes 20 and 20A becomes possible. Furthermore, it is not necessary to prepare a high voltage or a high pressure gas, and a burden on the facility management is alleviated.

In the cryotherapy devices 1 and 1A according to the embodiments described above, a combination of the target temperature of the probes 20 and 20A and the time length for which the target temperature is to be sustained is determined in advance, as at least two operation conditions. With this, appropriate treatment can be provided depending on the condition of the diseased cells.

In addition, in the cryotherapy devices 1 and 1A according to the embodiments described above, the control by the control units 4 and 4A is executed on the basis of the operation condition designated by the user. As a result, it is possible to prevent a situation in which appropriate treatment is not provided due to the user misrecognizing the appropriate time length for which the target temperature of the probes 20 and 20A is to be sustained.

In the cryotherapy device 1 according to the embodiment described above, the active surface 200 is surrounded by the surface 220 of the thermal insulation portion 22. As a result, normal cells around the tissue that is in contact with the active surface 200 are protected more reliably. In addition, because the surface 220 is flush with the active surface 200, the operation of bringing the active surface 200 into contact with the skin is not hindered by the presence of the thermal insulation portion 22.

In the cryotherapy device 1A according to the embodiment described above, in the probe 20A, the pair of surfaces 201A and 202A intersecting the active surface 200A is cooled by the pair of cooling elements 30A, instead of causing the cooling elements to cool the surface that is in parallel with the active surface. As a result, while it is possible to reduce restrictions in providing the active surface 200A, thereby making it easier to reduce the area of the active surface 200A, it is possible to position surfaces having relatively large areas, among the surfaces of the probes 20A, in a manner facing the surfaces capable of absorbing heat of the cooling elements 30A. In this manner, the probe 20A can be cooled via the two relatively large surfaces, so that the cooling efficiency can be further enhanced.

In the cryotherapy device 1A according to the embodiment described above, the pair of cooling elements 30A is sandwiched between the pair of cooling jackets 31A each having a substantially plate-like shape, with the probe 20A at the center. As a result, the cooling efficiency of the pair of cooling elements 30A can be further enhanced. In the head unit 2A, the probe 20A, the pair of cooling elements 30A, and the pair of cooling jackets 31A each of which has a substantially plate-like outer shape are stacked on top of one another. Thus, it is possible to keep the size of the entire head unit 2A relatively compact.

In the cryotherapy device 1A according to the embodiment described above, it is possible to confirm the condition of the skin to be treated on the display 9A using the imaging device 25A as necessary. With this, the operability of the head unit 2A is improved.

### <4. Modifications>

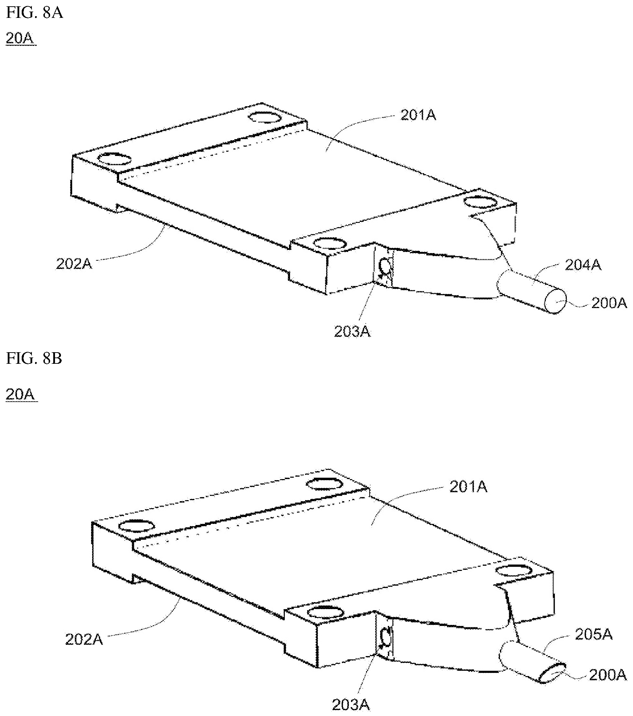

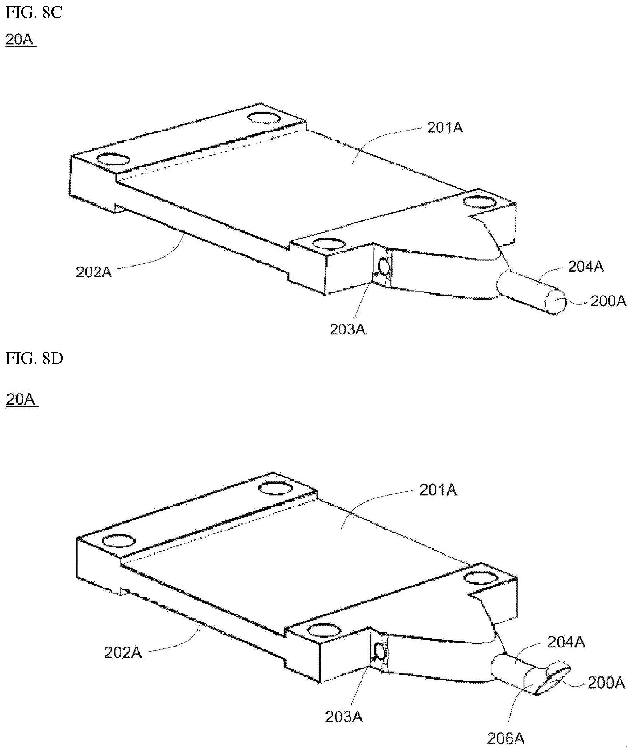

Although one embodiment of the present disclosure has been described above, the present disclosure is not limited to the embodiment described above, and various modifications are still possible within the scope not departing from the gist thereof. For example, the following modifications are still possible. The gist of the following modifications may be combined as appropriate.
(1) In the embodiments described above, the first target temperature of -30°C or above and -25°C or below and the second target temperature of -4°C or above and 0°C or below are described as target temperatures of the operation conditions stored in the storage device 44. However, the range of the target temperature is not limited thereto, and may be changed as appropriate. Even when the target temperatures of the probes 20 and 20A are predetermined temperatures of -25°C or above and -4°C or below, it is possible to exert an effect of selectively causing apoptosis in diseased cells including nevus cells, in the same manner as in the first target temperature and the second target temperature. In other words, the target temperature of the probe 20 may be any target temperature of -30°C or above and 0°C or below. As the time length for which the target temperature is to be sustained, an appropriate time length may be determined on the basis of the target temperature, as appropriate. In other words, the operation conditions stored in advance in the storage device 44 may be three or more, or there may be an operation condition in which the target temperature is the same but the time length for which the target temperature is to be sustained is different. Note that the cryotherapy devices 1 and 1A may be configured such that the user designates at least one of an appropriate target temperature and time length for which the target temperature is to be sustained to the control units 4 and 4A, without defining any particular operation conditions as described above.
(2) At least one of the temperature sensor 21 and the thermal insulation portion 22 may be omitted. Similarly, the illumination 24 is not essential, and may be omitted, or the location where the illumination 24 is positioned and the number of the illuminations 24 may be changed. It is possible for the surface 220 of the thermal insulation portion 22 not to be flush with the active surface 200. For example, the thermal insulation portion 22 in the surface 220 may protrude toward the skin surface with respect to the active surface 200 so that, when the surface 220 is brought into contact with the skin surface, the active surface 200 is positioned near the skin surface with a certain space with respect to the skin surface. Furthermore, at least one of the condition designation unit 5, the display unit 8, and the storage device 44 may be omitted, or may be configured separately from the housing where the control unit 4 is housed. The same is applicable to the condition designation unit 5A, the display unit 8A, and the control unit 4A. Similarly to the casing 23A, the casing 23 may also include a member enabled to fix the imaging device 25A.
(3) The cooling mechanism 3 may include two or more Peltier elements as the cooling elements 30. Possible alternatives for the Peltier elements serving as the cooling elements 30 and 30A are heat exchanging elements that undergo thermionic emission cooling when a current is passed, for example.
(4) The configurations of the cooling mechanisms 3 and 3A may be changed as appropriate, without limitation to the configurations according to the embodiments described above, as long as the temperatures of the probes 20 and 20A can be controlled therewith. For example, at least one of the cooling elements 30 and 30A, the heat-releasing member 31 and the cooling jackets 31A, and the chillers 32 and 32A may be omitted or replaced with another configuration. For example, the probes 20 and 20A may be cooled by a low-temperature liquefied gas or the like. Furthermore, the chillers 32 and 32A may be water-cooled. Furthermore, the cooling jackets 31A may be configured not as the pair of cooling jackets 3 1A A but as one cooling jacket 31A that cools the entire pair of cooling elements 30A.
(5) The probe 20 and the active surface 200, and the probe 20A and the active surface 200A may be made of a material other than metal. In addition, the probe 20 itself may be formed of a cooling element. Furthermore, the active surface 200 and the active surface 200A may be configured to be in proximity to the surface of the skin or to be in indirect contact with the surface of the skin with another substance therebetween, without limitation coming into direct contact with the skin surface of the treatment site.
(6) At least one of the temperature sensor 21A, the thermal insulation material 22A, the cover 231A, and the imaging device 25A may be omitted. Furthermore, the imaging device 25A is not limited to a camera, and may be, for example, an imaging device such as an ultrasonic echo device.
(7) The configuration of the probe 20A is not limited to that described in the embodiment. For example, the shape of the active surface 200A of the probe 20A and the elements therearound may be changed as illustrated in Figs. 8A to 8D. Fig. 8A illustrates an example in which a columnar portion 204A having a substantially columnar shape is provided to the distal end of the probe 20A illustrated in Fig. 6A. In this example, the circular flat surface on the distal end of the columnar portion 204A serves as the active surface 200A extending orthogonally to the pair of surfaces 201A and 202A. Fig. 8B illustrates an example in which a columnar portion 205A having a substantially truncated columnar shape is provided to the distal end of the probe 20A illustrated in Fig. 6A. In this example, the surface on the distal end of the columnar portion 205A serves as the active surface 200A. The active surface 200A is inclined with respect to the central axis of the columnar portion 205A. With the exemplary configuration illustrated in Figs. 8A and 8B, it is possible to bring the active surface 200A closer to or into contact with the skin tissue more easily. Fig. 8C illustrates an example in which a part of the surface on the distal end of the columnar portion 204A in Fig. 8A is a spherical surface. In this example, a surface excluding the perimeter of a part of the spherical surface (a boundary between a part of the spherical surface and the columnar portion) serves as the active surface 200A. With the exemplary configuration illustrated in Fig. 8C, in addition to being able to bring the active surface 200A closer to or into contact with the skin tissue more easily, the risk of physically damaging the skin tissue with the active surface 200A can be further reduced. Fig. 8D illustrates an example including an extended portion 206A that extends from the surface of the distal end of the columnar portion 204A in Fig. 8A in a manner inclined with respect to the central axis of the columnar portion 204A, and forms a flat surface on the distal end. The flat surface of the extended portion 206A serves as the active surface 200A. The extended portion 206A may have any configuration as long as the shape of the projection thereof as viewed from the direction of the central axis of the columnar portion 204A is different from the shape of the projection of the columnar portion 204A, and the active surface 200A intersects with the pair of surfaces 201A and 202A. With the exemplary configuration illustrated in Fig. 8D, the degree of freedom in the design of the active surface 200A is further increased, and the field of view is less obstructed by the probe 20A when the probe 20A is moved closer or brought into contact with the skin surface. In the exemplary configurations illustrated in Figs. 8A to 8D, the columnar portion 204A or 205A may be changed to a shape such as a substantially prismatic shape, a substantially oblique columnar shape, a substantially oblique prismatic shape, a substantially truncated conical shape, and a substantially truncated pyramidal shape. In addition, the probe 20A may be configured as one member, or may be configured by two or more members.

### Examples

An experiment carried out by the inventors and results thereof will now be explained. However, the present disclosure is not limited thereto.

### <Experiment>

A plurality of pieces of skin tissue were collected and placed on a hot plate heated approximately to 35°C. Observations were then made on the conditions of the cells in the skin tissue having been not subjected to any treatment (Comparative Example) and those having been subjected to the cooling treatment according to the embodiments described above, by bringing the active surface of the metal probe into direct contact with the surface of the skin tissue (Examples). Specifically, the pieces of skin tissue in Examples were subjected to the cooling treatment, and then the pieces of skin tissue in Examples and Comparative Example were both cultured for 12 hours, and then subjected to fixation treatment using a formalin solution. The pieces of skin tissue after the fixation treatment were stained using DAPI, which is a fluorescent dye that binds to DNA, and the fluorescent dye was then observed using a fluorescence microscope, so as to observe the cells included in the skin tissue (1). The pieces of skin tissue were also stained using MART-1, which is used for immunostaining melanoma and nevus cells, and nevus cells included in the skin tissue were then observed using a fluorescence microscope (2). In Example 3, however, the piece of skin tissue was stained using α-SMA, which selectively stains vascular smooth muscle, and the cells in a blood vessel were then observed, instead of nevus cells in the skin tissue (2'). The skin tissue was also stained using a marker that binds to DNA fragments resultant of apoptosis in accordance with the TUNEL method, and observations were made as to whether the cells in the skin tissue underwent apoptosis, using a fluorescence microscope (3). In the following drawings, micrographs resultant of capturing the images of the respective markers, using a fluorescence microscope, will be denoted as (1) to (3), respectively, and the superimposition resultant of superimposing the micrographs (1) to (3) will be denoted as (4). The micrographs (1) to (4) were obtained by capturing images of cross sections of the skin tissue, and the upper side corresponds to the epidermis side.

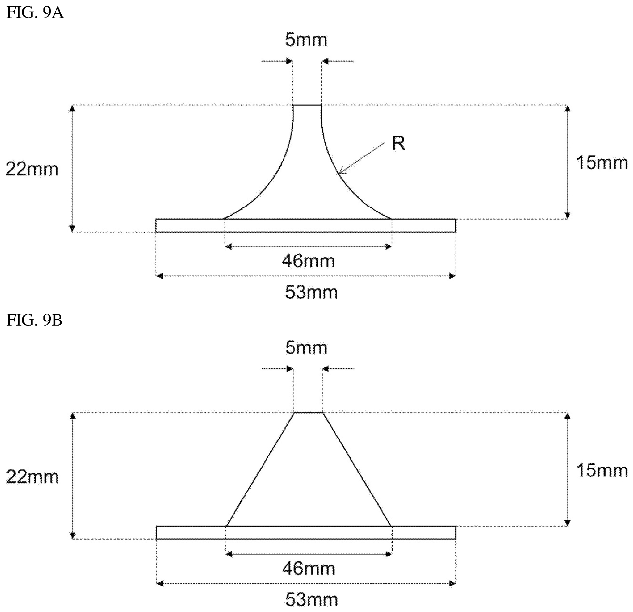

The cooling treatment was carried out under the following seven conditions. Fig. 9A is a side view of the probe used in Examples 1 to 6. As shown in Fig. 9A, the probe had a circular active surface having a diameter of 5 mm, and a circumferential side surface with an increasing diameter and extending from the perimeter of the active surface. The circumferential side surface delineated an R surface with a radius of 20 mm in the side view. As shown in Fig. 9B, the inventors confirmed that, through the experiment, the result from the probe with a substantially truncated conical shape with a linear circumferential side surface in the side view, and the result from the probe shown in Fig. 9A did not exhibit any significant difference in the change of dermis temperature with respect to the time. In Example 7, the probe (head unit) as illustrated in Figs. 6A and 6B was used. The probe had a circular active surface with a diameter of 5 mm. The temperature detected by the temperature sensor installed in the probe was used as the probe temperature, and exhibited a match with the temperature of the active surface. As the temperature sensor, any one of a Pt100 platinum resistance temperature detector, a K thermocouple, and a T thermocouple was used. In the probe illustrated in Fig. 9A, the temperature sensor was disposed at a distance of 7 mm from the active surface.
Example 1: Probe temperature: -25°C, length of time held in contact with skin tissue: 15 seconds
Example 2: With hair follicles in skin tissue, probe temperature: -25°C, length of time held in contact with skin tissue: 15 seconds
Example 3: With blood vessel in skin tissue, probe temperature: -25°C, length of time held in contact with skin tissue: 15 seconds
Example 4: Probe temperature: -4°C to -2°C, length of time held in contact with skin tissue: 60 seconds
Example 5: With hair follicles in skin tissue, probe temperature: -4°C to -2°C, length of time held in contact with skin tissue: 60 seconds
Example 6: With skin appendages (sebaceous glands) in skin tissue, probe temperature: -4°C to -2°C, length of time held in contact with skin tissue: 60 seconds
Example 7: Probe temperature: -25°C, length of time held in contact with skin tissue: 10 seconds

### <Results>

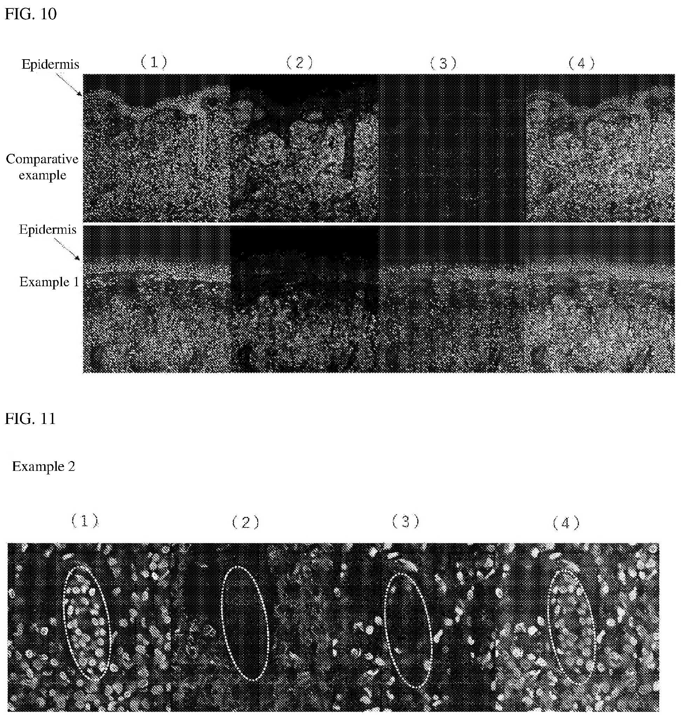

The micrographs obtained from the skin tissue in Examples 1 to 7 and Comparative Example are shown in Figs. 10 to 16. In Fig. 10, in the comparison of (3) between Comparative Example and Example 1, while the fluorescence of the marker was hardly visible in Comparative Example, the fluorescence of the marker was visible across a wide area in Example 1. The area where the fluorescence was confirmed substantially overlaps with the area where the nevus cells are present in (2). From these results, it was confirmed that apoptosis was induced in the nevus cells, as a result of the cooling treatment according to the embodiments described above.

The parts circled with broken lines in (1) to (4) of Fig. 11 correspond to where the hair follicles are present in the skin tissue. As can be seen from Fig. 11, a majority of the cells in the hair follicles was preserved without reaching apoptosis. From these results, it was confirmed that, as a result of the cooling treatment according to the embodiments described above, apoptosis was induced in the nevus cells while significantly suppressing the damage of hair follicle keratinocytes, which are normal cells. From these, it was confirmed that the cooling treatment according to the embodiments described above is capable of selectively inducing apoptosis in the nevus cells, without affecting hair follicles.

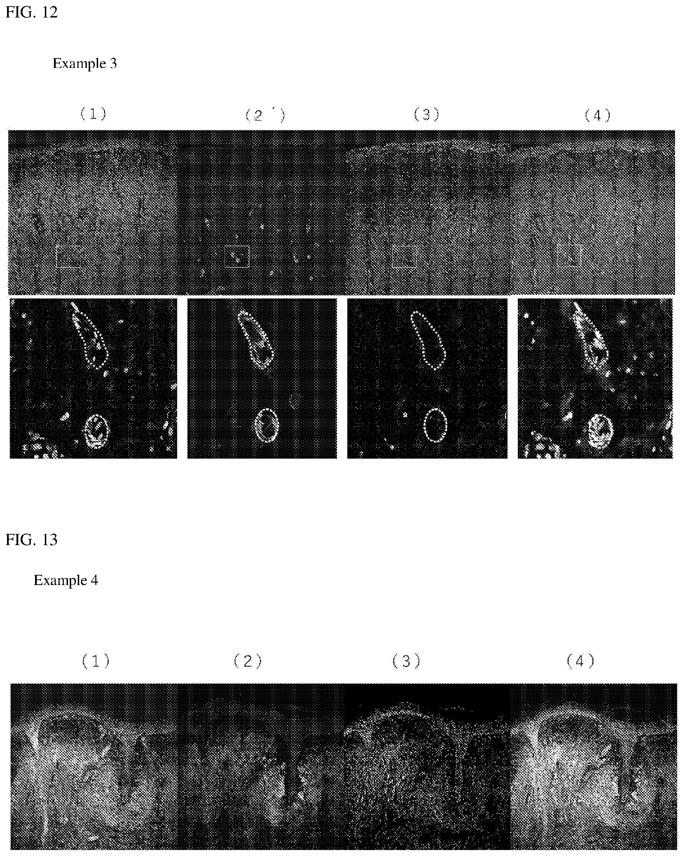

(1) to (4) of Fig. 12 are enlargements of rectangular portions surrounded by frames in the micrographs in the upper row, with the enlargements being shown in the micrographs in the lower row, respectively. The portions circled with white dotted lines in the lower micrographs (1) to (4) correspond to the cells forming a blood vessel. As can be seen from Fig. 12, a majority of the cells forming the blood vessels was preserved without reaching apoptosis. From these results, it was confirmed that the cooling treatment according to the embodiments described above hardly affected the blood vessels. From these, it was confirmed that the cooling treatment according to the embodiments described above is capable of selectively inducing apoptosis in the nevus cells, without affecting the blood vessels.

From Fig. 13, it was confirmed that apoptosis was induced in the area where the nevus cells were present. From these results, it was confirmed that, even when the temperature of the probe was as relatively high, e.g., -4°C to -2°C, the nevus cells were successfully led to apoptosis, by adjusting the time length of contact.

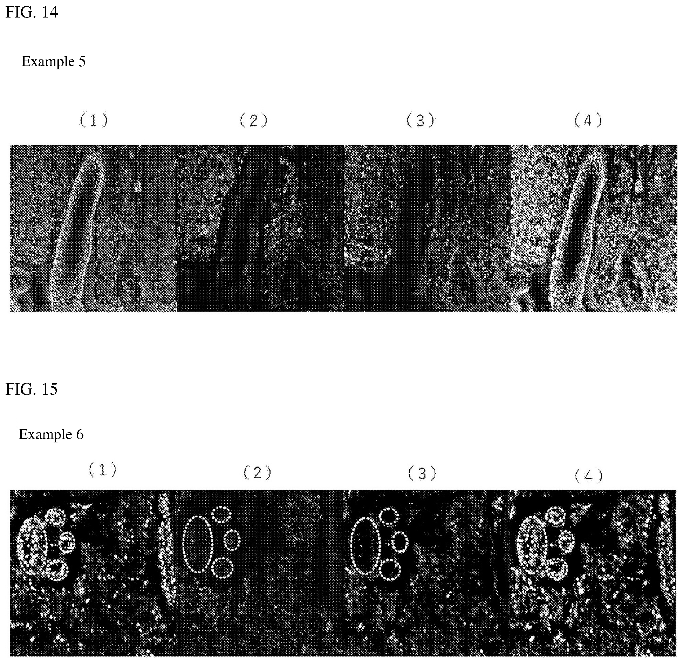

(1) to (4) of Fig. 14 are micrographs of the skin tissue near hair follicles. As can be seen from Fig. 14, a majority of the cells in the hair follicles was preserved without reaching apoptosis. From these results, it was confirmed that, even when the temperature of the probe was as relatively high as -4°C to -2°C, apoptosis was selectively induced in the nevus cells, while significantly suppressing the damage of hair follicle keratinocytes, which are normal cells.

The parts surrounded by white dotted lines in (1) to (4) of Fig. 15 correspond to sebaceous glands in the skin tissue. As can be seen from Fig. 15, a majority of the cells forming the sebaceous glands was preserved without reaching apoptosis. From these results, it was confirmed that, in the cooling treatment according to the embodiments described above, apoptosis was selectively induced in the nevus cells, while significantly suppressing the damage of skin appendages, which are normal cells.

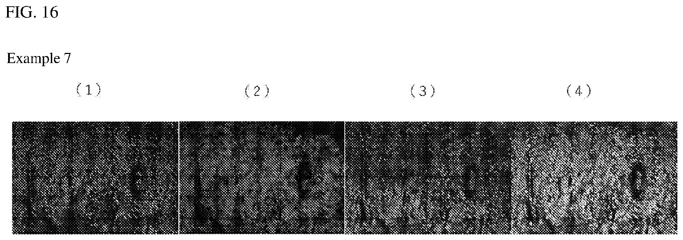

In Fig. 16, it was confirmed that apoptosis was induced in the region where the presence of nevus cells was observable. In particular, because a large amount of fluorescence was confirmed in the lower region of the image in (3), it was confirmed that apoptosis was induced into the deep nevus cells.

From the results of Examples 1 to 7, it was found that, although apoptosis could also be induced in some proportion of keratinocytes (epidermal keratinocytes), it was confirmed that such damage of normal cells was limited to the level of epidermis, and the risk of leaving scars and the like was sufficiently low.

### Reference Signs List

1, 1A Cryotherapy device
2, 2A Head unit
3, 3A Cooling mechanism
4, 4A Control unit
5, 5A Condition designation unit
20, 20A Probe
21, 21A Temperature sensor
22 Thermal insulation portion
22A Thermal insulation member
30, 30A Cooling element
31 Heat-releasing member
31A Cooling jacket
220 Surface

## Claims

1. A cryotherapy device comprising:
a head unit that includes a probe forming an active surface to be brought closer or into contact with a surface of skin of a treatment site;
a cooling mechanism that cools the probe; and
a control unit that controls the cooling mechanism so that the probe is sustained at a target temperature of 0°C or below,
wherein the probe is configured to freeze tissue of the skin via the active surface so as to selectively cause apoptosis in a diseased cell including at least a nevus cell.

2. The cryotherapy device according to claim 1, wherein
the probe further forms a pair of surfaces intersecting with the active surface,
the cooling mechanism includes a pair of cooling elements capable of cooling the probe, and
each of the pair of cooling elements forms a first surface capable of absorbing heat from the pair of surfaces.

3. The cryotherapy device according to claim 2, wherein each of the pair of cooling elements further forms a second surface capable of releasing the heat absorbed from the first surface, and the cooling mechanism further includes a cooling jacket through which a refrigerant capable of absorbing heat of the second surface is circulated.

4. The cryotherapy device according to claim 3, wherein the head unit further includes: a casing that houses at least a part of each of the probe, the pair of cooling elements, and the cooling jacket;
and a thermal insulation material disposed between the casing and the cooling jacket.

5. The cryotherapy device according to claim 1, wherein the head unit further includes an imaging device that acquires an image of the diseased cell including at least a nevus cell or vicinity of the diseased cell.

6. The cryotherapy device according to any one of claims 1 to 5, wherein the control unit controls the cooling mechanism so that the probe is sustained at a target temperature of -30°C or above.

7. The cryotherapy device according to claim 1 or 2, wherein the control unit controls the cooling mechanism so that the probe is sustained at a first target temperature of -30°C or above and -25°C or below.

8. The cryotherapy device according to claim 7, wherein the control unit controls the cooling mechanism to keep a time length for which the temperature of the probe is sustained at the first target temperature equal to or shorter than a first time length.

9. The cryotherapy device according to claim 1 or 2, wherein the control unit controls the cooling mechanism so that the probe is sustained at a second target temperature of -4°C or above and 0°C or below.

10. The cryotherapy device according to claim 9, wherein the control unit controls the cooling mechanism to keep a time length for which the temperature of the probe is sustained at the second target temperature equal to or shorter than a second time length.

11. The cryotherapy device according to any one of claims 1 to 5, wherein the head unit includes a temperature sensor that detects a temperature of the probe, and
the control unit feedback-controls the temperature of the probe based on an output signal from the temperature sensor.

12. The cryotherapy device according to claim 1, wherein the cooling mechanism includes a cooling element that cools the probe, and a chiller that cools the cooling element, and
the probe, the cooling element, and the chiller are connected in an order listed herein, with reference to the active surface.

13. The cryotherapy device according to any one of claims 1 to 5, wherein
the control unit controls the cooling mechanism in accordance with at least two different operation conditions each including a preset combination of a target temperature for the probe and a time length for which the target temperature is to be sustained, and
the cryotherapy device further comprises a condition designation unit that allows a user to designate one of the at least two different operation conditions.
